# EUROPEAN PATENT SPECIFICATION

(11) **EP 0 901 521 B1**
(45) Date of publication and mention of the grant of the patent: **02.11.2005**
(21) Application number: 97901754.8
(22) Date of filing: 03.02.1997
(51) Int. Cl.: C12N 5/08, A61K 35/18, A61K 35/12

(54) **ANTIGENIC MODULATION OF CELLS**
ANTIGENE MODULATION VON ZELLEN
MODULATION ANTIGENIQUE DE CELLULES

(30) Priority: 01.02.1996 KR 9602440; 27.06.1996 US 671452
(43) Date of publication of application: 17.03.1999
(73) Proprietor: BIOMEDICAL FRONTIERS, INC., Minneapolis, MN 55414 (US)
(72) Inventor: BYUN, Si-Myung, Taejon-Kwangyoksi 305-345 (KR); EATON, John, Baylor College of Medicine, Houston, TX 77030-3498 (US); JEONG, Seong-Tae, So-gu, Taejon-Kwangyoski 302-223 (KR); SCOTT, Mark D., Albany, NY 12208 (US)
(74) Representative: Evans, Claire
(86) International application number: PCT/IB1997/000139
(87) International publication number: WO 1997/028254

(56) References cited:
- WO-A-86/04145
- WO-A-92/05801
- WO-A-93/18649
- WO-A-95/26740
- J BIOMATER SCI POLYM ED 4 (6). 1993. 579-589, XP000673687 HAN D K ET AL: "PREPARATION AND SURFACE PROPERTIES OF PEO -SULFONATE GRAFTED POLYURETHANES FOR ENHANCED BLOOD COMPATIBILITY."
- ARTIF. CELLS, BLOOD SUBSTITUTES, IMMOBILIZATION BIOTECHNOL. (1996), 24(5), 503-511, 1996, XP000673685 JEONG, SEONG TAE ET AL: "Decreased agglutinability of methoxy-polyethylene glycol attached red blood cells: Significance as a blood substitute"

## Description

### Field of the Invention

The present invention relates generally to antigenic modulation of cells, and more particularly to non-immunogenic cellular compositions comprising cells modified with a non-immunogenic compound, and uses of such non-immunogenic cells.

### Background of the Invention

Throughout this application various publications are referenced, many in parenthesis. Full citations for these publications are provided at the end of the Detailed Description.

Acute tissue rejection can be observed in two major clinical situations: 1) blood transfusions; and 2) organ transplantation. In both situations, to be described in greater detail below, antibody binding and complement fixation are the two major mechanisms underlying the destruction of the donor tissue (the donor tissue referring to blood or organs). Previous means of attempting to control acute rejection have centered on tissue matching and pharmacologic interventions. Despite these measures a significant number of often life-threatening acute tissue rejection reactions continue to occur.

Blood transfusions are a crucial component in the treatment of a number of acute and chronic medical problems. These range from massive blood loss following traumatic injury to chronic transfusions to treat diseases such as thalassemia and sickle cell anemia. In most acute injuries simple blood typing (ABO/rh) is sufficient to identify appropriate donors. Occasionally, however, rare blood types are encountered where an appropriate match cannot be quickly found, a situation which may be life-threatening. More often problems are encountered in individuals, usually minorities, receiving chronic transfusions (e.g., as in sickle cell anemia and the thalassemias). Often, simple blood typing becomes insufficient in determining a proper match because these individuals develop transfusion reactions to minor red blood cell antigens. The transfusion reactions to these minor red blood cell antigens can make it nearly impossible to identify appropriate blood donors (Vichinsky et al. 1990).

To date, the only solutions to the above situations are to store autologous blood (frozen or at 4°C), keep a blood bank registry of potential donors with rare blood types, and to encourage minority blood donations. While all of these steps are prudent and variably effective, situations still arise where an appropriate (or even satisfactory) blood match cannot be made. Therefore, a need exists for methods and agents which will disguise otherwise immunogenic (or directly immunologically recognizable) red blood cells.

Similarly, the transplantation of organs (such as kidneys and livers) from one human to another is often made difficult by a lack of exact immunologic identify between donor and recipient. Sometimes, the transplanted organ is subject to direct attack by the immune system of the recipient even before a secondary immunologic response has had time to occur. This so-called 'hyperacute rejection' is often life threatening and, obviously, prevents the effective integration of the transplant into the recipient. Therefore, a need exists for methods and agents which may prevent immediate recognition of the endothelial surfaces of organ transplants, thereby moderating or stopping the process of acute graft rejection. In a similar vein, the transplantation of organs from one species to another ("xenotransplantation") faces even more formidable immunologic barriers and would be greatly facilitated by methods for blocking immunologic recognition of the foreign endothelial surface.

Proteins have been modified by the covalent attachment of soluble polymers such as polyvinyl alcohol, carboxymethyl cellulose (Mitz and Summaria 1961), and polyvinylpyrrolidone (von Spect et al. 1973). Various purified antigenic proteins have also been modified by covalent attachment of polyethylene glycols (PEGs) to render the resulting proteins non-immunogenic. Abuchowski et al. (1977a) disclose the modification of purified bovine serum albumin (BSA) by covalent attachment of methoxypolyethylene glycol, rendering the BSA non-immunogenic. Abuchowski et al. (1977b) disclose the modification of purified bovine liver catalase by covalent attachment of methoxypolyethylene glycol, rendering the catalase non-immunogenic. Jackson et al. (1987) disclose the modification of purified ovalbumin with monomethoxypolyethylene glycol using cyanuric chloride as a coupling agent. The resulting ovalbumin is non-immunogenic. Various reports have also shown that polyethylene glycol (PEG) coated liposomes have improved circulation time (Klivanov et al. 1991; Senior et al. 1991; Maruyama et al. 1992; and Lasic 1992).

Islets of Langerhans have been microencapsulated in semipermeable membranes in order to decrease immunogenicity of implanted islets (Lacy et al. 1991; Lim 1980). Sawhney et al. (1994) coated rat islets with a polyethylene glycol tetraarylate hydrogel. Importantly, PEG was not directly incorporated into the islet cell membranes but rather the cells were surrounded by the PEG-containing hydrogel.

Zalipsky and Lee (1992) discuss the use of functionalized polyethylene glycols for modification of polypeptides, while Merrill (1992) and Park and Wan Kim (1992) both disclose protein modification with polyethylene oxide.

U.S. Patent No. 4,179,337 of Davis et al. discloses purified polypeptides, such as enzymes and insulin, which are coupled to polyethylene glycol or polypropylene glycol having a molecular weight of 500 to 20,000 daltons to provide a physiologically active non-immunogenic water soluble polypeptide composition. The polyethylene glycol or polypropylene glycol protect the polypeptide from loss of activity and the composition can be injected into the mammalian circulatory system with substantially no immunogenic response.

U.S. Patent No. 5,006,333 of Saifer et al. discloses a biologically persistent, water-soluble, substantially non-immunogenic, substantially non-antigenic conjugate of superoxide dismutase, prepared by coupling purified superoxide dismutase to one to five strands of a polyalkylene glycol which is polyethylene glycol or polyethylene-polypropylene glycol copolymer, wherein the polyalkylene glycol has an average molecular weight of about 35,000-1,000,000.

U.S. Patent No. 5,013,556 of Woodle et al. discloses a liposome composition which contains between 1-20 mole percent of an amphipathic lipid derivatized with a polyalkylether, as exemplified by phosphatidylethanolamine derivatized with polyethylene glycol.

U.S. Patent No. 5,214,131 of Sano et al. discloses a polyethylene glycol derivative, a purified peptide modified by the polyethylene glycol derivative, and a method for production thereof. The polyethylene glycol derivative is capable of modifying the guanidine groups in peptides. The peptides modified by the polyethylene glycol derivative are extremely stable, are considerably delayed in biological clearance, and retain their physiological activities over a long period.

A need continues to exist for methods of making entire cells and tissues and organs, as opposed to purified proteins or peptides, non-immunogenic.

### Summary of the Invention

The invention provides a method for modulating the antigenicity and aggregation of mammalian, preferably human, cells. To this end, the subject invention provides for the covalent binding of a non-immunogenic compound to intact cells. Cells that can be effectively modified in accord with the invention include anucleate cells (platelets and red blood cells) and nucleated cells (epithelial cells, endothelial cells, and lymphocytes). The non-immunogenic compound may be any non-toxic or physiologically acceptable polymeric material which can be covalently attached to the cell to make the cell antigenically silent. The non-immunogenic compound or material may be attached directly to or through linking moieties to the antigenic determinants or may simply mask the antigenic determinants without being attached to the cell surface through the antigenic determinants, or a proportion of the non-immunogenic compound may be attached directly or through linking moieties to the antigenic determinants and another proportion thereof may be attached directly or through linking moieties to the cell surface without being attached to the cell surface through the antigenic determinants. In one embodiment, the non-immunogenic compound is polyethylene glycol (PEG) or a derivative thereof. Potential applications for PEG modification of cells include: 1) PEG-derivatized red blood cells (RBC) to diminish transfusion reactions arising from mismatched blood, including transfusions into people difficult to match (because they have pre-existing antibodies to minor blood groups) or sensitization to minor blood group antigens due to chronic transfusions; 2) PEG-derivatization of the vascular endothelium of donor tissues prior to transplantation to prevent/ diminish acute tissue rejection; 3) implantation of PEG-derivatized cells to correct enzyme deficiencies, other inborn errors of metabolism, or other types of defective cellular functions, 4) transfusion of derivatized RBC into malaria-infected individuals to correct the accompanying acute anemia and prevent the infection of the transfused cells, and 5) transfusions into people of unknown blood groups who may even differ in major (e.g., ABO) blood groups from the donor. Unexpectedly, red blood cells modified by PEG have normal *in vitro* and *in vivo* survival when compared to control cells.

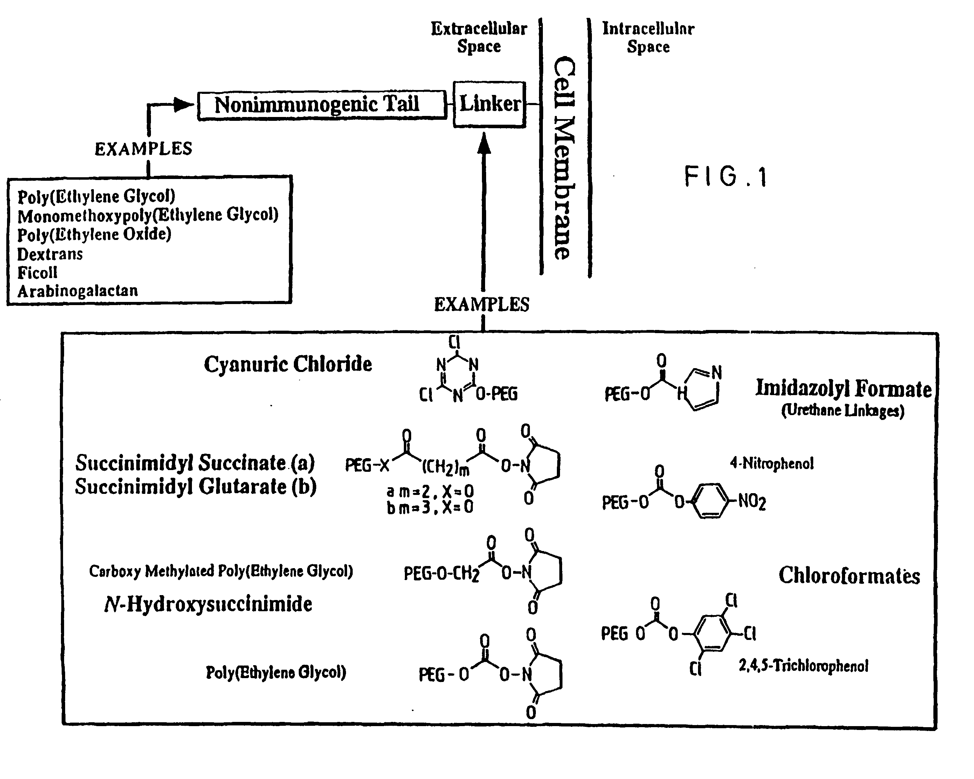

Covalent linkage of non-immunogenic compounds (e.g., PEG or PEG-derivatives, such a methoxypolyethylene glycol or PEG-like compounds such as polyethylene oxide), directly or indirectly to membrane proteins of cells decreases the antigenic recognition of these cells. Some of the available reactions and reagents to accomplish this are summarized in Figure 1. Similarly, insertion of PEG-modified phospholipids/free fatty acids into the cell membrane may serve a similar purpose. The examples hereinbelow-demonstrate that, unexpectedly, (1) it is possible to derivatize normal red blood cells and other cells with PEG, without lysis (2) that the derivatized red blood cells remain intact and exhibit normal morphology, (3) that PEG modification of the cell surface does, indeed, 'hide' antigenic determinants such as ABO blood groups, epithelial cell-specific antigens (ESA) and the MHC antigens which underlie tissue/organ rejection, (4) that the derivatized cells survive normally in the circulation of experimental animals, and (5) that PEG derivatized red blood cells from one species have vastly improved survival in the circulation of an animal from another species.

As delineated above, transfusion reactions (to both major and minor red blood cell antigens) represent a significant clinical problem. In most cases, these transfusion reactions actually result from minor surface antigens not routinely measured by blood banks. In situations where either an appropriate blood type match cannot be located or, more often, when sensitization to minor red blood cell antigens has occurred, PEG-modified red blood cells can be employed to diminish/prevent the recognition of red blood cell antigenic determinants, the application of this invention can also lead to procedures for modification of animal red blood cells which can then be used for transfusion into humans, or into animals of the same or other species. The application of this invention can further lead to procedures for modification of red blood cells to prevent malarial invasion or opsonization by factors such as complement.

In addition, based on the data contained in this disclosure, the scope of this invention extends well beyond blood banking to other areas where foreign tissues are manipulated or introduced *in vitro* or *in vivo.* One area of primary interest is the use of PEG-modified tissues (especially covalent modification of the vascular endothelium) for tissue transplantation. Despite appropriate HLA-matches, many organ transplants fail as a result of immediate tissue rejection. This rejection reaction occurs primarily at the level of the vascular endothelium and results in vessel occlusion, tissue hypoxia/ischemia and ultimate loss of the organ transplant. Based on the chemistry of PEG-cell derivatization disclosed herein, it is possible to perfuse the vasculature of the tissue with a solution of activated PEG. This will modify the vessel walls (i.e., endothelial cells) which will prevent or diminish the aforementioned immediate tissue rejection. This technology can thus improve the rate of successful tissue engraftment.

The invention thus provides a non-immunogenic cellular composition comprising: a cell having a cell surface and antigenic determinants on the cell surface; and a non-immunogenic compound covalently attached to the cell surface directly or by means of the linking moiety, which linking moiety can be derived from a linker molecule, as discussed below. The non-immunogenic compound acts to block recognition of the antigenic determinants on the cell surface. In one embodiment, the linking moiety is covalently.attached directly to the antigenic determinant on the cell surface. In an alternative embodiment, the linking moiety may be covalently attached to a non-antigenic site of the cell surface, the antigenic site on the cell surface is camouflaged or masked by virtue of the long chain length of the non-immunogenic compound.

The invention further provides a method of producing a non-immunogenic cell. The method comprises: covalently attaching a non-immunogenic compound to the surface of the cell directly, or by means of a linking moiety, so that non-immunogenic compound blocks recognition of antigenic determinants on the cell surface to produce a non-immunogenic cell. A non-immunogenic cell produced by this method is also provided by the subject invention.

The concept of the subject invention can also provide a method of decreasing phagocytosis or complement-mediated destruction or cell-mediated destruction of a cell. This method comprises: selecting a cell for introduction into a subject, the cell having a cell surface and antigenic determinants on the cell surface; covalently attaching an amount of a non-immunogenic compound to the cell surface directly or by means of a linking moiety, so that the attached non-immunogenic compound blocks recognition of antigenic determinants on the cell surface to produce a non-immunogenic cell; and introducing the non-immunogenic cells into a subject, wherein phagocytosis or complement- mediated destruction or cell-mediated destruction of the non-immunogenic cell is decreased as compared to phagocytosis or complement-mediated destruction or cell-mediated destruction of the cell prior to modification.

Destruction of cells can follow from phagocytosis or can follow from attachment of specific immunoglobulins to the antigenic determinants on the cell followed by a complement-mediated lysis without the participation of phagocytic cells. By blocking antibody binding *via* the covalent binding of the non-immunogenic compound or materials, not only phagocytosis is blocked but also complement-mediated lysis and other types of cell-mediated destructions.

Further provided is a method of decreasing an adverse reaction to a transfusion, the method comprising: selecting a red blood cell for transfusion into a subject, the red blood cell having cell surface and blood group antigenic determinants on the cell surface; covalently attaching a non-immunogenic compound capable of blocking the blood group antigenic determinants on the cell surface, to the cell surface directly or by means of a linking moiety, so as to produce a non-immunogenic red blood cell; and transfusing a subject with the non-immunogenic red blood cell, wherein adverse reaction to the transfusion of the non-immunogenic red blood cell is decreased as compared to transfusion of the red blood cell prior to modification.

Also provided is a method of decreasing rejection of a transplanted cell, the method comprising: selecting a cell for transplantation into a subject, the cell having a cell surface and antigenic determinants on the cell surface; covalently attaching a non-immunogenic compound capable of blocking the recognition of the antigenic determinants on the cell surface, to the cell surface directly or by means of a linking moiety, so as to produce a non-immunogenic cell; and transplanting the non-immunogenic cell into a subject, wherein rejection of the transplanted cell is decreased as compared to rejection of the cell prior to modification.

The invention provides a method of decreasing aggregation of nucleated and anucleate cells such as that induced by antibodies or by other cell:cell interactions. The method comprises: covalently attaching non-immunogenic compounds capable of blocking recognition of antigenic determinants on a cell surface to the cell surface of each of a plurality of cells directly or by means of a linking moiety, so as to produce non-aggregating cells, wherein antibody-induced aggregation of the non-aggregating cells is decreased as compared to antibody-induced aggregation of the cells prior to modification.

As used herein, the term "linking moiety" or "linker" refers to an at least divalent organic group that covalently, or by complexation or chelation binds to both the non-immunogenic molecule and the cell surface, to attach at least one non-immunogenic compound to at least one functional group or structure on the cell surface. The linking moieties can be derived from reactive linker molecules, as described hereinbelow.

### Brief Description of the Figures

These and other features and advantages of this invention will be evident from the following description of preferred embodiment when read in conjunction with the accompanying drawings in which:
Fig. 1 is a schematic depiction of the preparation of certain embodiments of the non-immunogenic cellular compositions according to the subject invention;
Fig. 2 is a schematic depiction of a further embodiment of a non-immunogenic cellular composition according to the subject invention. In this embodiment, the non-immunogenic compound is polyethylene glycol or a derivative thereof and the activated PEG (PEG-linker) is covalently attached to antigenic determinants on the cell surface (directly blocking antigenic sites) and also covalently attached to non-antigenic sites on the cell surface (indirectly blocking antigenic sites due to their long chain length);
Fig. 3 is a graph showing that monomethoxypoly(ethylene glycol) (mPEG) modification of red blood cells causes a dose-dependent inhibition of anti-A antibody induced RBC aggregation defined turbidometrically;
Fig. 4 is a bar graph showing that mPEG modification of red blood cells only slightly increases red blood cell lysis;
Fig. 5 is a graph showing the mPEG modification of red blood cells has no effect on red blood cell osmotic fragility;
Fig. 6 is a bar graph showing that mPEG-modified type A red blood cells bind significantly less anti-A antibody;
Fig. 7 is a bar graph showing that mPEG-modified sheep red blood cells are significantly less prone to phagocytosis by human peripheral blood monocytes;
Fig. 8 is a graph showing no significant differences in the *in vivo* survival of control mouse red blood cells and mouse red blood cells modified with activated PEG; and
Fig. 9 is a graph demonstrating that sheep red blood cells (solid symbols) enter and survive within the circulatory system of a mouse whereas unmodified sheep red blood cells (open symbols) do not.
Fig. 10 is a graph depicting the Donor A PBMC (peripheral blood mononuclear cells) response to antigenically foreign Donor B PBMC (Panel A) and the Donor B PBMC response to Donor A (Panel B) in MLC analysis of control and mPEG derivatized PBMC.
Fig. 11 is a graph demonstrating that platelet aggregation is prevented by the covalent modification of platelet surfaces with mPEG.
Fig. 12 is a bar graph demonstrating that mPEG-modification of epithelial cells blocks antibody recognition of surface antigens.
Fig. 13 is a graph showing oxygen dissociation curves of unmodified red blood cells and red blood cells modified with mPEG.

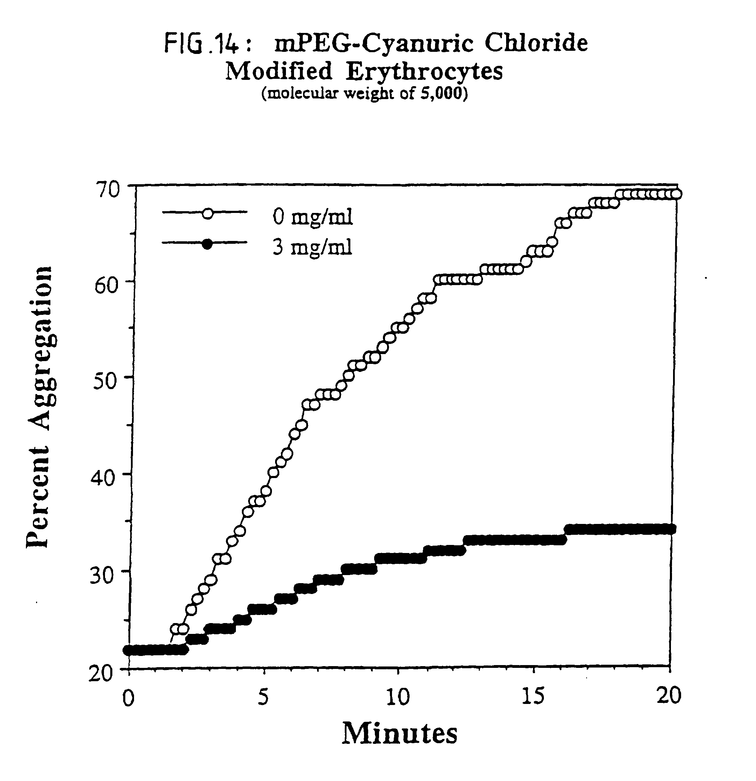

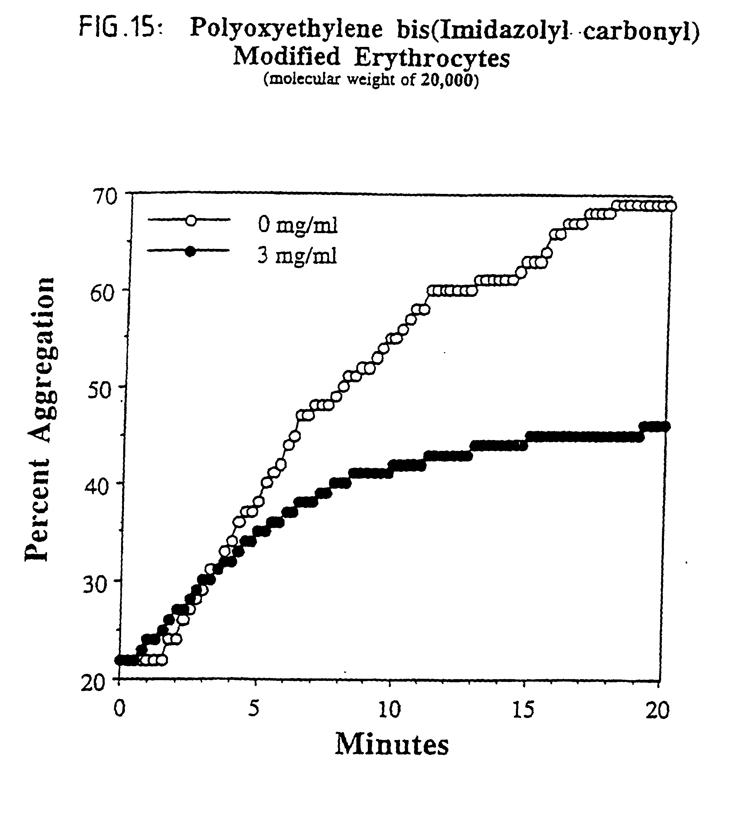

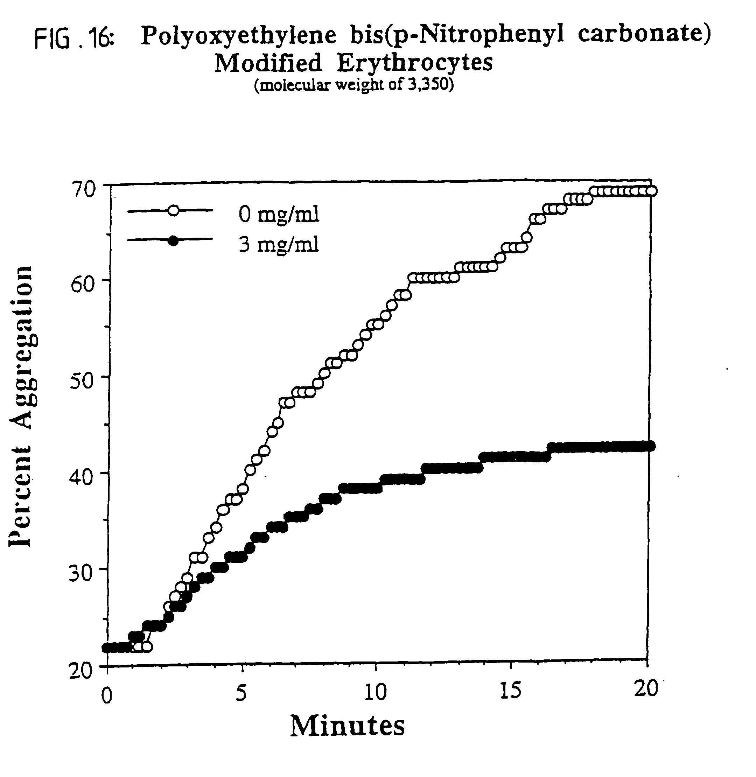

Figs. 14 to 16 are graphs showing the effects of functionalized polyethylene glycols or derivatives thereof on typing sera-induced erythrocyte aggregation.

### Detailed Description

The present invention provides a non-immunogenic cellular composition comprising: a cell having a cell surface and antigenic determinants on the cell surface; a linking moiety covalently attached to the cell surface; and a non-immunogenic compound covalently attached to the linking moiety and capable of blocking recognition of the antigenic determinants on the cell surface. Alternatively, the non-immunogenic compound can be bound directly to the cell surface, if it comprises groups such as carboxylic acids, aldehydes, ketals or acetals that are reactive with NH₂ or SH groups on the cell surface.

The cell can be any suitable cell with accessible antigenic determinants on the cell's surface. Suitable cells include anuclear cells, for example, hematopoietic cells, i.e., red blood cells or platelets, or nucleated cells, for example, vascular endothelial cells, PBMCs, hepatic cells, neuronal cells, pancreatic cells, or epithelial cells.

The antigenic determinants on the cell surface can be due to the presence of antigenic proteins, antigenic carbohydrates, antigenic sugars, antigenic lipids, antigenic glycolipids, antigenic glycoproteins, etc. "Antigenic" determinants can also be involved in malarial invasion of a cell, or opsonization of a cell. For example, red blood cells have antigens on their surface which determine ABO/rh blood types. These antigens are often referred to as blood group antigenic determinants. These antigens are recognized by an incompatible host and the donor cell will be rapidly destroyed. This can involve the enhancement of natural immunity (through phagocytes, such as macrophages, neutrophals, and natural killer cells) or the stimulation of specific or acquired immunity (including humoral immunity through antibodies and cell-mediated immunity through T lymphocytes). In any event, the cell is recognized as foreign and elicits an immune response.

In order to prevent this immune response from destroying the cell, the subject invention involves modification of the antigenicity of the cell. This modification is accomplished by attaching a non-immunogenic compound to the cell. Suitable non-immunogenic compounds for use in the subject invention are non-immunogenic compounds capable of blocking recognition of antigenic determinants on the cell surface. The compounds are generally long chain compounds, wherein the long chain can sterically block the antigenic determinants. Such non-immunogenic compounds include polyalkylene glycols such as polyethylene glycol, polypropylene glycol, mixed polypropylene-polyethylene glycols, or derivatives thereof (including methoxypolyethylene glycol), certain polysaccharides such as dextrans, cellulosics, Ficoll, and arabinogalactan, and nontoxic, hydrophillic, synthetic polymers, including polyurethanes. Useful molecular weights of these compounds can range from about 100-500 to 100,000-200,000 or above.

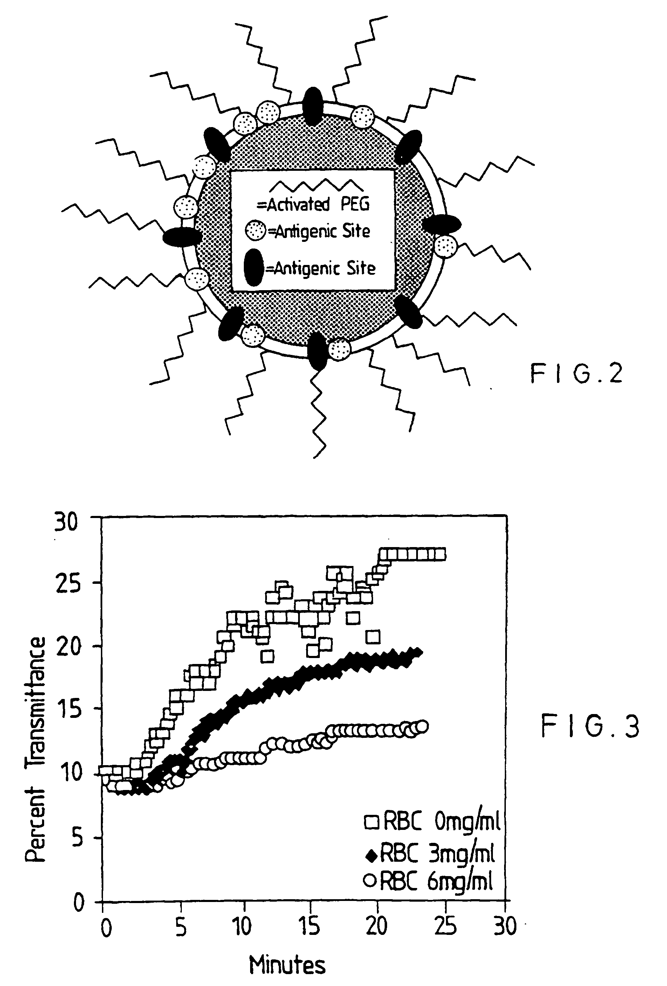

The presently preferred non-immunogenic compound according to the subject invention is polyethylene glycol or a derivative thereof. The polyethylene glycol or derivative thereof is a molecule with a very long chain length. The non-immunogenic compound (e.g., polyethylene glycol or derivative thereof) can be directly attached to an antigenic site (e.g., an antigenic determinant) on a cell surface via a linking moiety (direct modification of antigenicity) (see Fig. 1 and Fig. 2) or can be attached to a non-antigenic site on the cell surface via a linking moiety. In both cases, the long chain of the non-immunogenic compound (e.g., polyethylene glycol or derivative thereof) effectively blocks antigenic sites on the cell surface (indirect modification of antigenicity) (see Fig. 2). In either embodiment, the non-immunogenic compound (e.g., polyethylene glycol or derivative thereof) is attached to the cell surface by a linking moiety, which is derived from a linker molecule that can react with the PEG. The combination of a polyethylene glycol or derivative thereof and the linker molecule is generally referred to as an "activated" polyethylene glycol or derivative thereof.

Polyethylene glycols (PEG) and derivatives thereof are well known in the art. Polyethylene glycol has the formula

H(OCH₂CH₂)ₙOH

wherein n is greater than or equal to 4, with a molecular weight of up to about 20,000 Daltons. However, polyethylene glycol and derivatives thereof are available having molecular weights of up to about 200,000 or more, and are also useful in the practice of the invention, alone or in combination with lower m.w. materials. Various derivatives of polyethylene glycol comprise substitutes for the H or OH end groups, forming, for example, polyethylene glycol ethers (such as PEG-O-R; PEG-O-CH₃; CH₃-PEG-OH or "mPEG"; 2,4-dinitrophenyl ethers of PEG), polyethylene glycol esters (such as PEG-O₂C(CH₂)₁₄CH₃; PEG-O₂CCH₂CH₂CO₂-atropine), polyethylene glycol amides (such as PEG-O₂C(CH₂)₇CONHR; mPEG-O₂CCH₂CH₂CONH(CH₃)CHCH₂C₆H₅; PEG-O₂CCH₂CH₂CONHCH₂CH₂-NAD⁺), polyethylene glycol amines (such as PEG-NH₂; PEG-NH(CH₂)₆NH,; PEG-OCH₂CH₂NH₂; mPEG-NH₂), polyethylene glycol acids (such as PEG-O₂C(CH₂)₂CO₂H; PEG-OCH₂CO₂H; PEG-O₂C(CH₂)₇-CO₂H), polyethylene glycol aldehydes (PEG-O-CH₂-CHO), and electrophilic derivatives (such as PEG-Br; PET-OSO₂CH₃; PEG-OTs). Various phenyl moieties can also be substituted for the H or OH of PEG, such as the 2,4-dinitrophenyl ether of PEG mentioned above). Methoxypolyethylene glycol (m.w. 2,000-8,000) is a preferred PEG derivative for use in the present invention.

For a full discussion of polyethylene glycol and activated derivatives thereof, including the synthesis of the derivatives, see the following references: Harris et al. 1984; Harris 1985; Zalipsky and Lee 1992; Park and Kim 1992; Merrill 1992; and U.S. Patent Nos. 4,179,337 and 5,214,131, the contents of each of which are incorporated herein by reference. The particular non-immunogenic compounds, including the polyethylene glycol derivatives, listed above are exemplary only, and the invention is not intended to be limited to those particular examples.

According to the subject invention, these non-immunogenic compounds (e.g., polyethylene glycol molecules or derivatives thereof) are covalently attached to the cell surface by means of a linking moiety. These linking moieties can be prepared by reaction of the polyethylene glycol or derivative thereof with suitable linker molecules that are also well known in the art, and include, for example, cyanuric chloride, imidazolyl formate, succinimidyl succinate, succinimidyl glutarate, N-hydroxysuccinimide, 4-nitrophenol, and 2,4,5-trichlorophenol. These linker molecules "activate" the PEG, a term also well known in the art. For a description of activation of PEG, with examples of known linking moieties and molecules, see Harris 1985. The linker molecules listed above are exemplary only, and the invention is not intended to be limited to those particular examples. As would be recognized by one of skill in the art, the linking molecules disclosed hereinabove and on Figure 1 react with a reactive group such as a hydroxy of the non-immunogenic compound, e.g., the PEG or MPEG, and also react with an NH₂ or, in some cases, SH, group of a peptidyl or other amino acid residue on the cell surface to covalently join them, whereby the linking molecule is converted in one or more steps into a divalent linking moiety such as shown on Table 1, below.

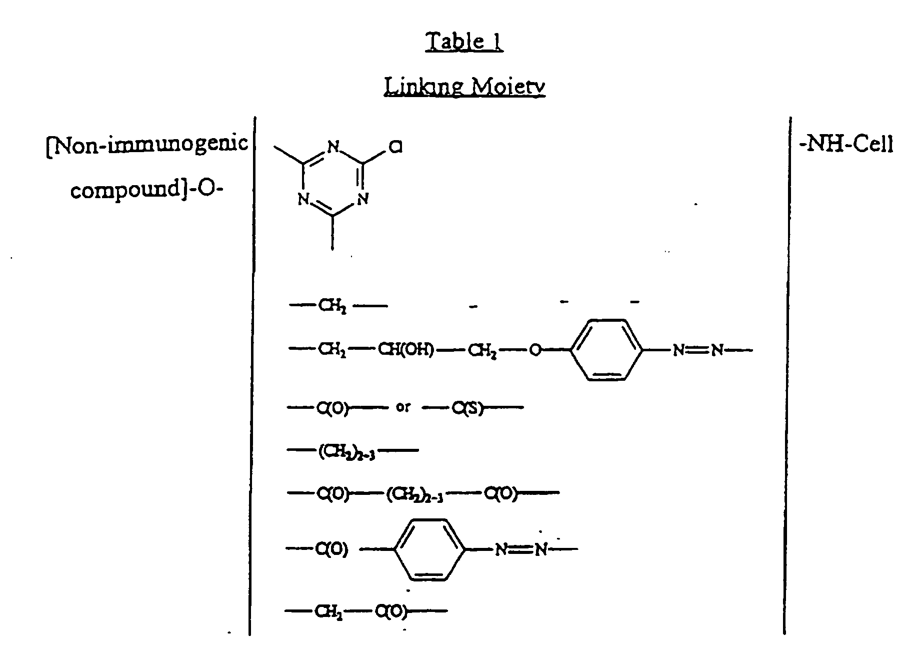

A number of "activated" methoxypolyethylene glycols are commercially available, in which mPEG (m.w. 5000) has been bound to a linking molecule at the hydroxyl terminus. These include, methoxypolyethylene glycol (mPEG) para-nitrophenyl carbonate, mPEG cyanuric chloride, mPEG-succinimidyl succinate, mPEG tresylate, and mPEG imidazolyl carbonyl. For example, see I. Jackson et al., Anal. Biochem., 565, 114 (1987); A. Abuchowski et al., J. Biol. Chem., 252, 3578 (1977); F. M. Veronese et al., Appl. Biochem. Biotech., 11, 141 (1985), C. Delgado et al., Biotech. Appl. Biochem., 12, 119 (1990); C. O. Veavchemp et al., Anal. Biochem., 131, 25 (1983).

The chemistry involved in the covalent attachment of the non-immunogenic compound (such as PEG or a derivative thereof) to reactive groups such as proteins and peptides on the cell surface (thus, covalent attachment of the non-immunogenic compound to a cell surface) by means of linking moieties, is known in the art, and is discussed in detail in Harris 1985; Harris et al. 1984; and Zalipsky and Lee 1992.

Preferably, for the chemical modification of the red blood cells, the free hydroxyl group at one end of the mPEG was activated with cyanuric chloride and reacted with hemoglobin to confirm activation. Next, human or animal derived red cells were diluted with 0.9% NaCl aqueous solution, preferably to approximately 2% (v/v), and a NaOH solution was slowly added using pH titrimetry, maintaining pH at about 9 to 10, preferably at 9.2. 1 mM to 10 mM mPEG, activated as disclosed above, was added to the 2% (v/v) red cell solution, which was then reacted under ambient temperatures while being mixed slowly. After the reaction was finished, the red blood cells were precipitated in a centrifuge, suspended with PBS (phosphate buffered saline, pH 7.4) and centrifuged again. The final red blood cells were obtained by repeating this precipitation and suspension process. This process can be used with human or animal derived red blood cells, even those with an expired storage period. Also, the pH 9.2 borate buffer used in conventional mPEG chemical modification was not used. Instead, pH titrimetry was used to adjust the pH of the red cell solution diluted with an aqueous solution of 0.9% NaCl.

A blood group antigen-antibody reaction slide test and microwell precipitation test were conducted to verify whether red cells chemically modified in this way can be used for transfusion regardless of blood type. The slide test is a method generally used to verify blood type before transfusion. In this method, red blood cells and anti-A, anti-B and anti-D (Rh) antibodies, the red blood cells' blood group antibodies, are mixed on a slide and observed for agglutination. As a result of employing this method, a reduction of agglutinability was observed in red blood cells chemically modified with mPEG.

In addition, agglutination of red blood cells on microwell plates and binding with blood group antibodies were tested to quantifiably verify the reduction of agglutination. When each well on a microwell plate is filled with a different concentration of blood group antibodies and a fixed concentration of chemically modified red cells, the red cells react with the blood group antibodies, gradually precipitating them. The degree of red cell agglutination was quantified as the final concentration of blood group antibodies showing no aggregation of red blood cells at the center of the wells. Red blood cells treated with various concentrations of mPEG were initially reacted with blood group antigens, then centrifuged to obtain a supernatant fluid. This was then reacted with red blood cells that had not been chemically modified and the number of blood group antibodies remaining in the supernatant fluid was quantified. As a result, it was found that chemically modified red blood cells exhibited reduced agglutinability and that binding with blood group antibodies was suppressed.

From the above results showing a connection between blood group antibody binding and reduction in agglutinability, it was found that the agglutination of red blood cells is reduced by mPEG, which obstructs the binding of blood group antibodies with red blood cells. To determine how this reduction of agglutinability is triggered on the surface of the red blood cells, the membranes of chemically modified red blood cells were separated and subjected to 12% SDS-PAGE and staining with Coomassie blue or periodic acid. The membrane protein of the ABH antigen groups, band 3, 4.5, PAS-1 and PAS-2 were analyzed. As a result, it was found that as the concentration of processed mPEG increased, bands 3 and 4.5, and bands PAS-1 and PAS-2 gradually disappeared, showing gradual deposition in the upper portions of the gel. This shift of bands is a result of the direct bonding of mPEG with the membrane protein of red blood cells. It can be seen, therefore, that mPEG reduces the agglutinability of red cell antigens by obstructing the access of antibodies through its covalent binding to the antigen portions of these membrane proteins or the areas around them.

To verify whether chemically modified red blood cells have oxygen transport activity sufficient for their use in transfusions, an oxygen dissociation curve was calculated, analyzing the degree of hemoglobin binding with oxygen in the red cells, based on oxygen partial pressure. Chemically modified red blood cells exhibited an oxygen dissociation curve form, oxygen affinity, a P₅₀ value, an n value (the Hill coefficient indicating cooperative effect) and oxygen transport to tissues that were similar to those of unmodified red blood cells. It was found, therefore, that binding of mPEG to red blood cells does not cause a change in oxygen transport activity.

Red blood cells chemically modified in accordance with the present invention, therefore, can be used regardless of blood type and have sufficient oxygen transport activity. As a result, they can be used in all medical fields in which red blood cells are used, for such purposes as emergency blood transfusion and transplant organ storage. The method of producing red blood cells chemically modified in accordance with the present invention can be applied to all blood group antigens regardless of blood type. The method is very simple and economical, making it very advantageous for the design of reactors for mass production in the future.

The invention thus further provides a method of producing a non-immunogenic cell. The method comprises: covalently attaching a non-immunogenic compound capable of blocking recognition of antigenic determinants on a cell surface, to a cell surface, directly, or by means of a linking moiety, so as to produce a non-immunogenic cell. If the cell is a red blood cell, the method can further comprise transfusing a subject with the non-immunogenic cell. Since the antigenic determinants, such as the blood group antigenic determinants, on the red blood cell are blocked by the non-immunogenic compound, the transfused non-immunogenic red blood cell will not elicit an immune response. As discussed above, this method can be very useful when red blood cells need to be transfused quickly without the availability of complete blood typing or cross-matching, or when only unmatched blood from a subject is available.

If the cell is part of a tissue or organ, the method can further comprise transplanting the non-immunogenic tissue or organ into a subject. Since the antigenic determinants on the tissue or organ, such as the vascular endothelial cells which form an exposed antigenic surface of the tissue or organ, are blocked by the non-immunogenic compound, the transplanted non-immunogenic tissue or organ will not elicit an immune response. As discussed above, this method is very useful to avoid severe rejection reactions, or graft vs. host disease, when organs or tissues are transplanted.

The invention further provides a non-immunogenic cell produced by the above method.

The concept of the subject invention can also provide a method of decreasing phagocytosis of a cell. This method comprises: introducing the non-immunogenic cell into a subject, wherein phagocytosis of the non-immunogenic cell is decreased as compared to phagocytosis of the cell prior to modification. The non-immunogenic cell can be.prepared by a process comprising: selecting a cell for introduction into a subject, the cell having a cell surface and antigenic determinants on the cell surface; covalently attaching to the cell surface, directly or by means of a linking moiety, a non-immunogenic compound that blocks recognition of the antigenic determinants on the cell surface, so as to produce a non-immunogenic cell. In the case where the cell is a red blood cell, this method can prevent phagocytosis of the "foreign" red blood cell, by rendering the red blood cell non-immunogenic. The "foreign" red blood cell may be from another human, or may be from another non-human subject. In either case, the body's response would be to attempt to eliminate the "foreign" red blood cell including by phagocytosis.

Further provided is a method of decreasing an adverse reaction to a transfusion, the method comprising: transfusing a subject with the non-immunogenic red blood cell, wherein adverse reaction to the transfusion of the non-immunogenic red blood cell is decreased as compared to transfusion of the red blood cell prior to modification. The non-immunogenic red blood cells are prepared by selecting a red blood cell for transfusion into a subject, the red blood cell having a cell surface and blood group antigenic determinants on the cell surface; covalently attaching to the cell surface a non-immunogenic compound in an amount capable of blocking the blood group antigenic determinants on the cell surface; wherein the compound is covalently attached to the cell surface directly or by means of a linking moiety, so as to produce a non-immunogenic red blood cell. As discussed above, the red blood cell could be from another human or from a non-human mammal.

Also provided is a method of deceasing rejection of a transplanted cell, the method comprising: transplanting a non-immunogenic modified cell into a subject, wherein rejection of the transplanted modified cell is decreased as compared to rejection of the cell prior to modification. The cell is prepared by a process comprising: selecting a cell for transplantation into a subject, the cell having a cell surface and antigenic determinants on the cell surface; covalently attaching a non-immunogenic compound to the cell surface directly or by means of a linking moiety, so that the non-immunogenic compound blocks the recognition of the antigenic determinants on the cell surface, to produce a non-immunogenic cell. Where the cell is part of a tissue or organ which is to be transplanted into a subject, a preferred method of carrying out the covalent attachment is to perfuse the tissue or organ with a solution of an activated polyethylene glycol or derivative thereof (i.e., the polyethylene glycol or derivative thereof is first attached to the linker molecule, forming an activated PEG, which is then perfused over the tissue or organ). During the perfusion, the activated PEG covalently attaches to the cell surface via a linking moiety.

The invention provides a method of decreasing antibody-induced aggregation of cells, the method comprising: covalently attaching to the cell surface non-immunogenic compounds capable of blocking recognition of antigenic determinants on the cell surface; wherein the compounds are covalently attached to the cell surface of each of a plurality of cells, directly or by linking moieties, so as to produce non-aggregating cells, wherein antibody-induced aggregation of the non-aggregating cells is decreased as compared to antibody-induced aggregation of the cells prior to attachment of the compounds. This method is particularly applicable where the cells are red blood cells, and where the antigenic determinants on the cell surface comprise blood group antigenic determinants.

In each of the above-described methods, a linker molecule can be first reacted with the non-immunogenic compound (forming an "activated" compound) and then the linker molecule can be reacted with the cell surface. The order of these steps can be reversed, and any reference to the two steps is intended to cover the two steps in either order. Accordingly, the linker molecule can also be attached to the cell surface, then the non-immunogenic compound can be reacted with the linker molecule to bind it to the cell surface via a thus-formed linking moiety, in accordance with the claims and disclosure herein.

In the examples which follow, PEG modification of the external aspect of the red blood cell membrane effectively 'hides' major antigenic determinants such as ABO blood group substances. This is evident in the (1) lack of gross antibody-induced agglutination, (2) significantly decreased antibody-induced aggregation, and (3) diminished phagocytosis by heterologous macrophages. Treated red blood cells remain intact, exhibiting only minor spontaneous hemolysis, and demonstrate normal osmotic fragility over at least 48 hours *in vitro* incubation. The "normal" nature of the modified mouse red blood cell is further demonstrated by normal *in vivo* survival.

The PEG modification procedure is surprisingly well tolerated by the cells, yielding a product which survives normally in the circulation. The derivatized cells are antigenically disguised and not recognized by blood group antibodies or by phagocytes. Perhaps most surprisingly, treated red blood cells from one species survive much longer than do untreated red blood cells in the circulation of another species.

The invention thus provides for (1) derivatization of human red blood cells to permit transfusions into people difficult to match (because they have pre-existing antibodies to minor blood groups); (2) derivatization of human red blood cells to permit transfusions into people of unknown blood groups who may even differ in major (e.g., ABO) blood groups from the donor; (3) derivatization - by perfusion of activated mPEG solutions - of human organ grafts to prevent unexpected hyperacute rejection episodes; (4) derivatization - by perfusion of activated mPEG solutions - of organs from non-human animals to prevent hyperacute rejection and to improve the chances of ultimate successful engraftment in humans.

### EXAMPLE I

### Inhibition of Red Blood Cell Agglutination:

Normal human red blood cells (erythrocytes) were washed 3 x in isotonic saline. A red blood cell suspension of hematocrit about 12% is prepared in isotonic alkaline phosphate buffer (PBS; 50 mM K₂HPO4 and 105 mM NaCl, pH about 9.2). Cyanuric chloride-activated methoxypolyethylene glycol (Sigma Chemical Co.) is added and the red cells are incubated for 30 minutes at 4°C. Cell derivatization can also be done under other pH and temperature conditions with comparable results to those presented. For example, red blood cells derivatized at pH 8.0 for 60 minutes at 22°C demonstrated virtually identical characteristics to those derivatized at pH 9.2 for 30 minutes at 4°C. The extreme range ofpH and temperature conditions make this procedure broadly applicable to a wide range of cells and tissues. The proposed mechanism of covalent reaction with external proteins and other membrane components is outlined below. Typical activated mPEG concentrations used range from 0 to 8 mg per ml of red blood cell suspension. The typical activated mPEG concentration to be used on other anuclear (i.e., platelets) and various nucleated cells (e.g., vascular endothelial, hepatic, hematopoietic, neuronal, pancreatic cells, epithelial cells, etc.) can readily be determined in view of the teachings herein.

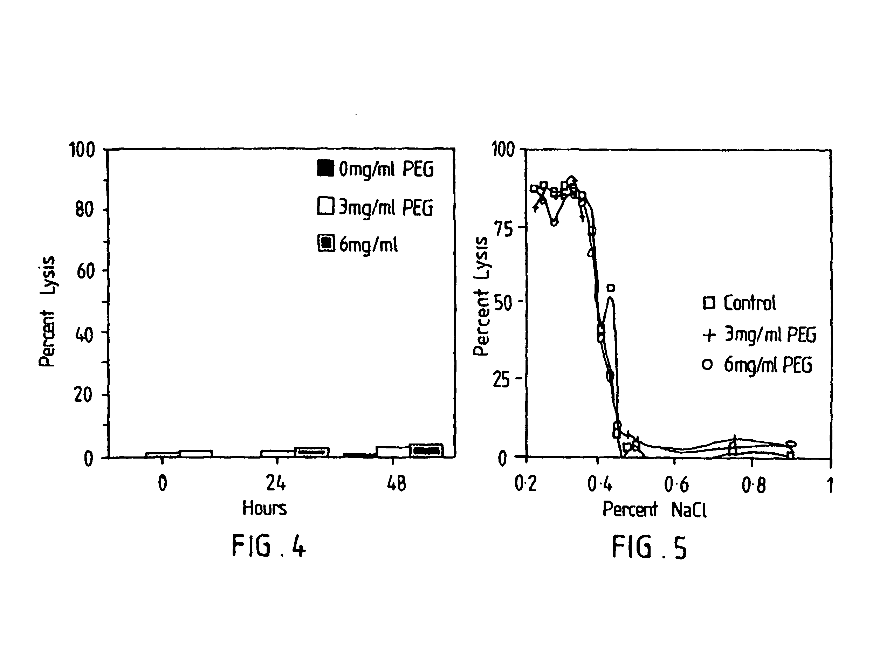

As shown in Figure 3, the covalent binding of mPEG to the membrane proteins of intact red blood cells prevents red blood cell agglutination. This is apparent at the gross level using agglutination induced by ABO antibodies, and at a finer level using a platelet aggregometer modified to measure red blood cell aggregation (Fig. 3). Type A red blood cells were treated with 0, 3, or 6 mg cyanuric chloride-activated mPEG (m.w. 5000) per ml of blood and incubated at 4°C for 30 minutes. The cells were washed 3 times with isotonic saline and resuspended to a 40% hematocrit in saline.

For gross agglutination, equal volume of a RBC suspension of hematocrit 40% and a commercially available anti-A blood typing antibody (Carolina Biological Supply) were mixed and photographed. Increasing amounts of bound mPEG effectively inhibited the agglutination reaction. In the absence of derivatization, a typical blood typing response was observed. In contrast, with increasing amounts of covalently bound mPEG, a dose-dependent decrease in sera induced agglutination of RBC was observed. Indeed, at 6 mg mPEG/ml RBC, no detectable agglutination was observed at the gross level.

Fig. 3 shows red blood cell microaggregation as measured at 37°C in a platelet aggregometer. As shown, mPEG modification caused a dose-dependent inhibition of anti-A antibody induced red blood cell aggregation.

Further testing of matched control and mPEG-derivatized RBC selected minor RBC antigens also demonstrated a significant decrease in the antigenicity of the mPEG-modified RBC (Table 2).

**Table 2**

| Detection of Selected Rh and MNS PBS Antigens on Control and Derivatized RBC | | | | | | | |
|---|---|---|---|---|---|---|---|
| **Antigen** | C | c | E | e | K | S | s |
| **Control** | 0 | 4⁺ | 0 | 3⁺ | 0 | 3⁺ | 3⁺ |
| **mPEG-Treated** | 0 | 1^{+w} | 0 | 1^{+w} | 0 | 1⁺ | 1^{+w} |

Agglutination response is measured macroscopically with a 4+^{s} rating being the strongest and 1 +^{w} being the weakest agglutination response. As shown, in all cases where a minor RBC antigen was detected, mPEG-modification virtually abolished its detection (e.g., 4⁺ to 1^{+w}). Importantly, the degree of activated mPEG derivatization used in this study was relatively low (6 mg/ml) in comparison to the levels which can be used (up to approximately 30 mg mPEG/ml RBC) while exhibiting no adverse effects on the RBC. Indeed, based on the mPEG-dose dependency noted in Fig. 3, it is very likely that higher degrees of derivatization will likely further suppress antigen detection.

### EXAMPLE II

### Effect on Red Blood Cell Stability:

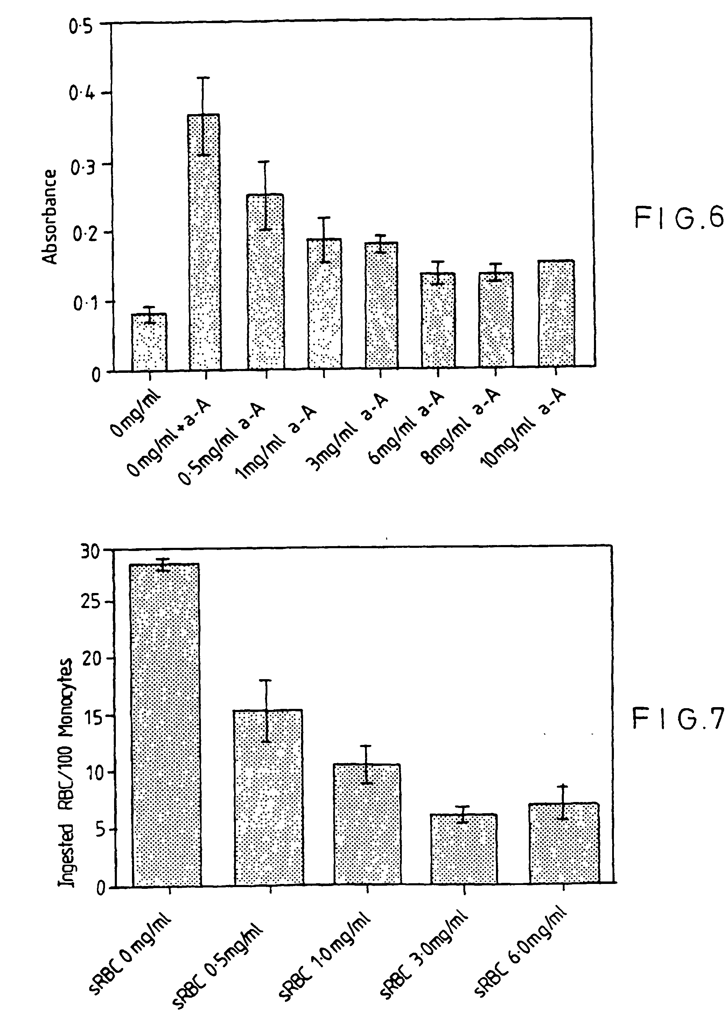

While mPEG-modification of red blood cells slightly increases red blood cell lysis, this lysis is less than 5% of the total red blood cell mass (Fig. 4). Furthermore, mPEG-attachment was found to have no effect on red blood cell osmotic fragility (Fig. 5). Red blood cell stability was minimally modified by the covalent attachment of mPEG. As shown in Fig. 4, red blood cell lysis was slightly increased by the attachment of mPEG. However, red blood cell lysis of the RBC during mPEG modification, followed by 24 hours storage at 4°C or after incubation at 37°C was less than 5%. As shown in Fig. 5, osmotic fragility of the mPEG-treated red blood cells was also unaffected. Shown are the osmotic fragility profiles of control and mPEG-modified (3 and 6 mg/ml) red blood cells after 48 hours incubation at 37°C. Again, while a very minor increase in spontaneous lysis was observed, no significance differences in the osmotic lysis profiles were seen. Electron micrographic analysis of control and mPEG-derivatized RBC also demonstrate no apparent structural changes.

### EXAMPLE III

### Inhibition of Antibody Binding:

mPEG-modified red blood cells bind significantly less anti-A antibody (Fig. 6). As shown in Fig. 6, an ELISA assay of mPEG-treated human blood type A⁻ red blood cells demonstrates significantly less antibody binding by mPEG-modified red blood cells. The control and mPEG red blood cells were mixed with an IgG anti-A antibody incubated for 30 minutes. The samples were extensively washed and a secondary antibody (anti-human IgG conjugated with alkaline phosphatase) was added to quantitate bound anti-Blood group A antibody.

### EXAMPLE IV

### Inhibition of Phagocytosis of Foreign Cells:

mPEG-modified sheep red blood cells are significantly less prone to phagocytosis by human peripheral blood monocytes (Fig. 7). As would be indicated by decreased antibody binding (Fig. 6), mPEG-modified sheep red blood cells are significantly less susceptible to IgG-mediated phagocytosis by human peripheral blood monocytes. mPEG-modified sheep red blood cells were incubated with human peripheral blood nzonocytic cells for 30 minutes. The uningested red blood cells were removed by hypotonic lysis and the number of monocytes containing sheep red blood cells, as well as the number of sheep red blood cells ingested, were determined microscopically.

### EXAMPLE V

### mPEG-Derivatized Mouse Red Blood Cells Have Normal In Vivo Survival:

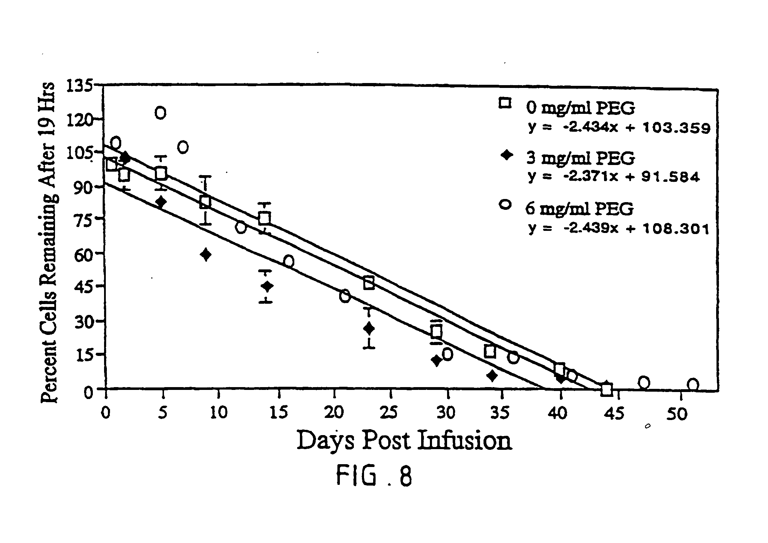

As shown in Fig. 8, no significant differences were noted in the *in vivo* survival of control red blood cells and red blood cells modified with either 3 or 6 mg/ml activated mPEG. *In vivo* survival of control and mPEG-modified mouse red blood cells was determined using a fluorescent fatty acid label (PKH-26; Sigma Chemical Company). Blood was obtained from donor BALB/C mice, treated with 0, 3, or 6 mg/ml activated mPEG and washed thrice. The washed cells were then labeled with PKH-26 and injected *i*.*p*. into naive BALB/C mice. Blood samples were obtained by tail-cuts at the indicated time points and analyzed via FACScan.

### EXAMPLE VI

### mPEG-Derivatization of Sheep Red Blood Cells Results in Enhanced In Vivo Survival in Mice:

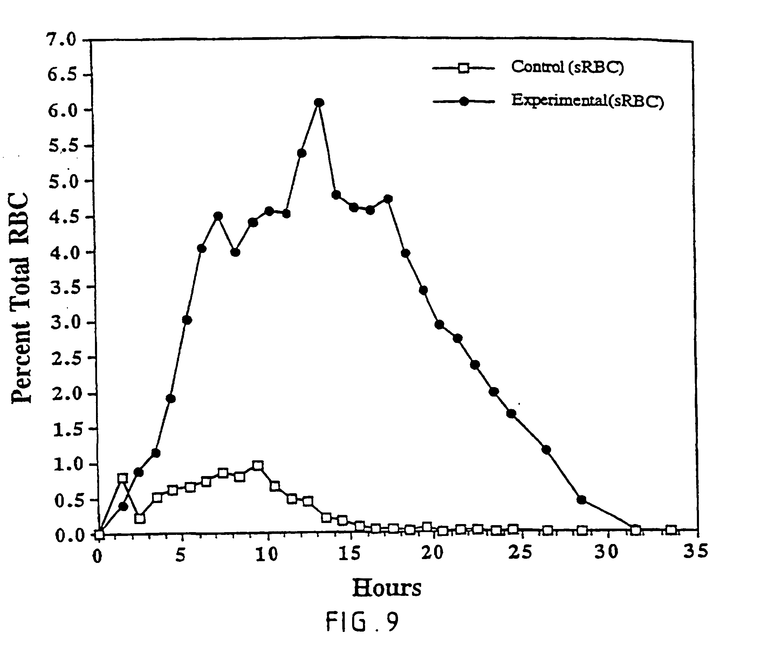

Comparable numbers of mPEG-modified sheep red blood cells (mPEG-sRBC) were injected *i*.*p*. into BALB/C mice. As shown in Fig. 9, mPEG-sRBC showed a greater rate of entry into the peripheral circulation and demonstrated longer *in vivo* survival in mice. *In vivo* survival of mPEG-sRBC in mice was determined using a fluorescent fatty acid label (PKH-26; Sigma Chemical Company). Blood was obtained from a donor sheep and treated with 0 or 6 mg/ml activated mPEG and washed thrice. The washed sheep red blood cells were labeled with PKH-26 and injected *i.p.* into naive BALB/C mice. Blood samples were obtained by tail-cuts at the indicated time points and analyzed via FACScan.

### EXAMPLE VII

### mPEG-Modulated Lymphocytes:

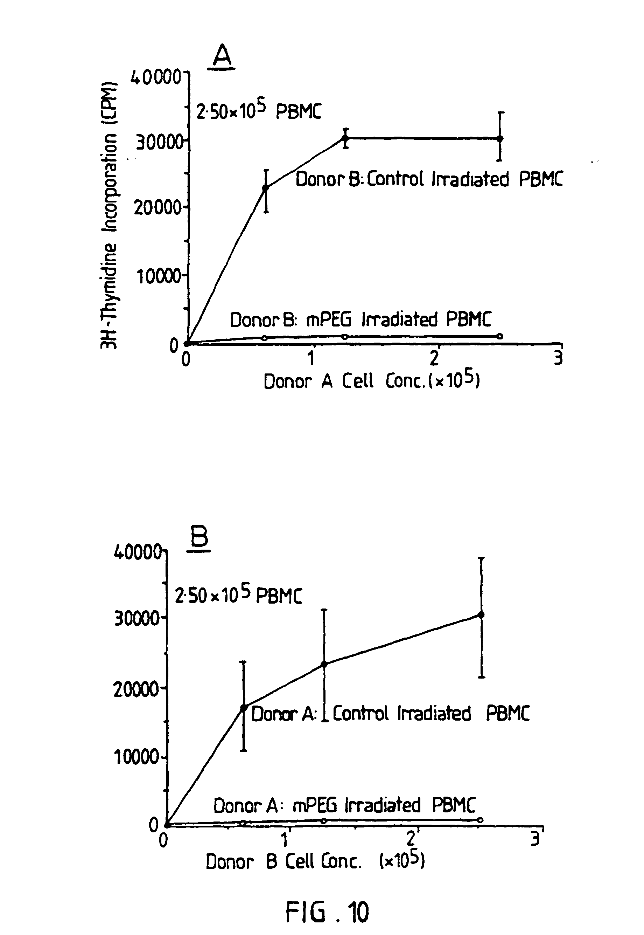

The mixed lymphocyte culture (MLC) is a very sensitive measure of histocompatibility between donor and recipient. Indeed, though time consuming, this assay is perhaps the best indicator of the probability of tissue transplant survival in the organ recipient. Primarily, the MLC measures the antigenic variance between the HLA complex (the primary antigens responsible for tissue compatibility in transplants) between two individuals. As shown in Figure 10, covalent modification with mPEG of lymphocytes from either donor results in a virtually complete inhibition of recognition of the antigenically foreign lymphocytes. Shown is the proliferation, measured by ³H-thymidine incorporation into DNA, of responder cells in response to a fixed concentration (2.5 x 10⁵ PBMC) of stimulator (i.e., cells irradiated to prevent cell replication). Panel A demonstrates PBMC Donor A's response to antigenically foreign Donor B PBMC. Panel B demonstrates Donor B's response to Donor A. In contrast, the population of responder (i.e., nonirradiated) cell expands tremendously in response to control irradiated PBMC (peripheral blood mononuclear cells).

These results are further confirmed by photomicrographs of the mixed lymphocyte cultures. Extensive proliferation, cell spreading, and expansive foci of responder cells are seen in response to control stimulator cells. In contrast, the same population of responder cells fails to recognize mPEG-treated stimulator cells, remain morphologically unactivated and fail to proliferate.

### EXAMPLE VIII

### Modification of Platelets:

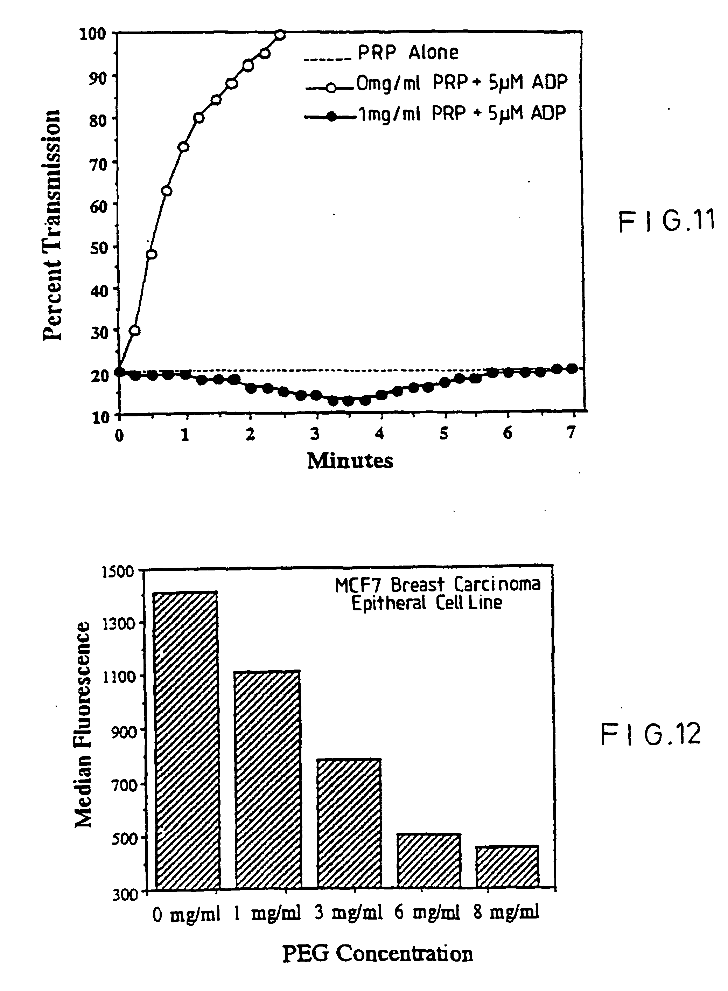

Other blood cells are also amenable to mPEG modification. Platelets were modified at pH 8.0 for 60 minutes at room temperature by the procedure of Example 1. The dotted line represents platelet rich plasma (PRP) in the absence of ADP (i.e., control unactivated platelets). As demonstrated in Figure 12, mPEG derivatized platelets do not aggregate in response to activation by ADP (5 µM). While control platelets are fully aggregated within approximately 2 minutes, mPEG-modified platelets remain unaggregated even after 7 minutes of exposure to ADP. The loss of aggregation is mediated by disruption of cell:cell interaction (i.e., preventing platelet interaction and microaggregate formation). Indeed, alteration in cell:cell interaction is a primary event due to the covalent modification of cell surfaces with non-immunogenic materials.

To determine if non-hematological cells could be antigenically modified by mPEG-derivatization, a breast carcinoma epithelial cell line (MCF7) was examined. A mouse monoclonal antibody directed towards epithelial specific antigen (ESA; a 40 kD glycoprotein) was chosen. Mouse anti-human ESA binding was quantitated using a BD-FACScan. FITC-conjugated goat anti-mouse antibody was used to detect bound ESA. Epithelial cell concentration was 5 x 10⁵ cells/ml with a 1:6000 titre of anit-ESA antibody. Epithelial cells were derivatized using a modification of the RBC-derivatization protocol. Specifically, confluent monolayers of MCF7 cells were scraped from tissue culture flasks and suspended in RPMI media. The cell suspensions were incubated with increasing concentrations of activated mPEG at pH 8.0 and incubated at room temperature for 60 minutes. The cells were then washed 3 x with culture media prior to the antibody binding assay.

As shown in Figure 12, a mPEG-dose dependent decrease in ESA-specific antibody binding was observed. At the highest mPEG dosage used (8 mg/ml cells) a > 70% decrease in anti-ESA binding was observed.

### EXAMPLE IX

### Separation of Red Blood Cells from Human and Animal Blood:

Sheep's blood (Korea Medical, Republic of Korea), the type of which was not distinguished from human type AB blood (Korea National Red Cross, Republic of Korea), was collected and centrifuged for five minutes at 1,100 x g. Plasma and white blood cells were separated, then the precipitate obtained was resuspended in a 0.9% NaCl isotonic solution (hereafter referred to as "saline") and centrifuged for five minutes at 120 x g. The red blood cells were washed by repeating this process five times. Finally, the solution was centrifuged for five minutes at 1,100 x g, the supernatant fluid was removed, and the resulting concentrated red blood cells (100% of saline (v/v)) were stored at 4°C.

### EXAMPLE X

### Chemical Modification of Red Blood Cells by mPEG:

To chemically modify the separated red blood cells, the mPEG's free hydroxyl group was activated as follows using cyanuric chloride as an activating agent. Twenty grams of mPEG were dissolved in 160 ml of anhydrous benzene heated to 70°C, then 5.6 g of cyanuric chloride and 4 g of anhydrous potassium carbonate were added, and the solution was slowly mixed and reacted in a vacuum desiccator at ambient temperature for 16 hours. Afterwards, the solution was filtered using a sintered glass filter and 200 ml of petroleum ether were added to the filtrate to precipitate the mPEG. The resulting white precipitate was again filtered using a sintered glass filter and dissolved by the addition of 150 ml of anhydrous benzene. After repeating this process more than six times to completely remove any unreacted cyanuric chloride, the activated mPEG was precipitated for the last time, desiccated under a vacuum, divided into 1 g portions, sealed to prevent penetration by moisture, and stored at -10°C.

To chemically modify red blood cells using the synthetic activated mPEG of Example X, human or sheep derived concentrated red cells produced as described in Example IX were diluted with saline and adjusted to 2% (v/v). Using pH titrimetry, a 0.5 M NaOH solution was slowly added to maintain pH at 9.2. Adding 0, 0.5, 1, 2, 3, 4 and 5 mM activated mPEG by concentration, the solution was slowly mixed and reacted for one hour at ambient temperature. When the reactions had terminated, the solution was centrifuged to precipitate the red blood cells, which were then suspended in PBS (phosphate buffered saline, pH 7.4) and centrifuged again. The final red cells were obtained by repeating this precipitation-suspension process three times. The cells were then diluted with saline to a concentration of 20% (v/v) and stored at 4°C.

### EXAMPLE XI

### Confirmation of Reduction in Agglutinability of Chemically Modified Red Blood Cells:

To determine whether chemically modified red blood cells can be used for transfusion regardless of blood type, a study was conducted on blood group antigen-antibody reactions using the following slide test and microwell precipitation test methods. The slide test is a method in which 100 µl each of anti-A, anti-B and anti-D (Rh) blood group antibodies are placed on a slide, followed by 50 µl each of 20% (v/v) human or sheep red cell sample, chemically modified as described in Example X, then slowly shaken by hand for approximately one minute, until agglutination is seen. The results were photographed as soon as they were visually identified. A reduction of agglutinability was observed in red cells treated with 3 mM of mPEG. An agglutination test using the red blood cells of sheep produced identical results.

A microwell agglutination test was used to quantify agglutination. It can identify the minimum concentration of antibodies triggering agglutination, because blood group antibodies are examined using serial dilution. The bottoms of the 96 microwells used in this test had a V shape, making it easy to identify the presence of agglutination during precipitation.

Anti-A, anti-B and anti-D (Rh) blood group antibodies were serially diluted by one-half and 70 µl of each was added to the wells. Twenty µl of the test sample of human red blood cells diluted at 1% (v/v) was added to each well and left for approximately two hours. The microwell plate was then examined at a magnification of 20 under an inverted microscope. The degree of agglutination of red blood cells was quantified as the final concentration (dilution ratio) of antibodies showing the state of precipitation in which no aggregates formed and red cells rolled down the walls, gathering on the bottoms of the microwells (see Table 3).

The microwell precipitation test (antibody binding test) was also used to determine the number of blood group antibodies binding to the surface of red blood cells. Into tubes containing 150 µl each of the blood group antibodies anti-A, anti-B, and anti-D (Rh) were added 40 µl of the test sample of red blood cells diluted at 1% (v/v). These were mixed thoroughly, then reacted for one hour, at ambient temperatures in the case of anti-A and anti-B blood group antibodies, at 37°C in the case of anti-D (Rh) blood group antibodies. Next, the initially reacted antibodies were removed by centrifuging; while the supernatant fluid was retained and subjected to one-half serial dilution in the microwells. Unmodified red blood cells were added to this fluid (1% (v/v)) and reacted. By using unmodified red blood cells, it was possible to measure the quantity of residual antibodies that had reacted in the initial reaction between the blood group antibodies and red blood cells (see Table 3). The data contained in Table 3, below, show quantities of blood group antibodies determined by using human red cells. Data from these agglutination tests using sheep red cells are not presented because identical results were obtained.

**Table 3**

| The extent of binding of blood group antibodies and agglutination in microwells of chemically modified red blood cells, expressed as the dilution ratio of blood group antibodies. | | | | | | |
|---|---|---|---|---|---|---|
| Table 1: Binding of Blood Group Antibodies and Agglutination of Chemically Modified Red Blood Cells | | | | | | |
| | Agglutination | | | Antibody Bonding | | |
| Type of Antibody | Anti-A | Anti-B | Anti-D (Rh) | Anti-A | Anti-B | Anti-D (Rh) |
| Unmodified Red Blood Cells | 2¹¹ | 2¹¹ | 2⁵ | 2¹ | 2⁴ | 2⁴ |
| Modified Red Blood Cells | | | | | | |
| 0 mM mPEG | 2⁹ | 2¹⁰ | 2⁴ | 2¹ | 2⁴ | 2⁴ |
| 0.5 mM mPEG | 2⁸ | 2⁸ | 2³ | 2² | 2⁶ | 2⁴ |
| 1 mM mPEG | 2⁷ | 2⁷ | 2² | 2³ | 2⁷ | 2⁴ |
| 2 mM mPEG | 2⁶ | 2⁶ | 2¹ | 2⁴ | 2⁸ | 2⁵ |
| 3 mM mPEG | 2⁵ | 2⁶ | 2¹ | 2⁸ | 2⁸ | 2⁵ |
| 4 mM mPEG | 2⁴ | 2⁶ | 2¹ | 2⁸ | 2⁹ | 2⁵ |
| 5 mM mPEG | 2⁴ | 2⁶ | 2¹ | 2⁸ | 2⁹ | 2⁵ |

As can be seen in Table 3, red blood cells chemically modified with mPEG have a final aggregate concentration considerably reduced compared with that of the unmodified controL From the much higher number of blood group antibodies remaining in the supernatant fluid, it was found that red blood cells chemically modified with mPEG have reduced agglutinability and suppressed binding of blood group antibodies. This means that the reduction of agglutinability was caused by the blood group antibodies' inability to bind with red blood cells due to the mPEG.

### EXAMPLE XII

### Examination of the Mechanism of Agglutinability Reduction in Chemically Modified Red Blood Cells:

Osmotic shock was used to separate the membrane of the chemically modified red cells described in Example XI and the membrane proteins were analyzed using 12% SDS-PAGE. In the case of the ABH antigen group, a membrane protein of the red cell conferring blood group antigenicity, antigens are generally reposed to be present in carbohydrates such as the glycophorin of band 3, band 4.5, PAS-1 and PAS-2. In the case of the Rh antigen group, antigens are reported to be present in the protein. Glycoprotein, an ABH antigen, does not stain well with the conventional method of protein staining, Coomassie blue, so PAS (periodic acid staining) was used.

As the concentration of modified mPEG increased, bands 3 and 4.5, and bands PAS-1 and PAS-2 gradually disappeared, showing gradual deposition in the upper portions of the gel. This shift of bands is a result of mPEG binding with the membrane protein of red blood cells. mPEG binding takes place only at the membrane surface and, therefore, does not react with bands 1 and 2, which contained spectrin, a protein from the membrane interior. No shift of these bands was observed. It was found, therefore, that mPEG bonded directly with these membrane proteins, causing a reduction in the agglutinability of red cell antigens. Results identical with those above were seen in an analysis conducted using the red blood cells of sheep.

### EXAMPLE XIII

### Confirmation of the Oxygen Transport Activity of Chemically Modified Red Blood Cells:

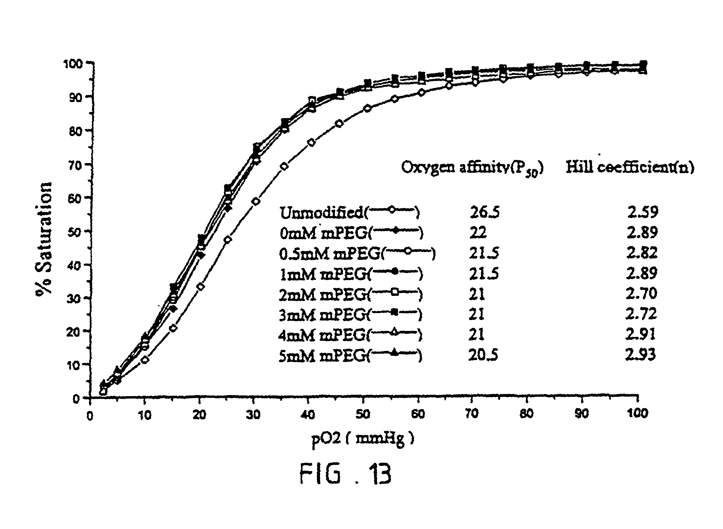

When it binds with oxygen, the 560 nm absorbency peak of hemoglobin in red blood cells divides into peaks of 540 nm and 576 nm. In the present invention, a Hemox Analyzer (TCS Medicals Co., USA), which automatically measures this change of absorbency, was used to generate an oxygen dissociation curve in accordance with the method used by Stètler et al., Bio/Technology, 9, 57 (1991). A sample for analysis was prepared by adding to a 4 ml Hemox buffer solution, 10 µl of antifoaming agent and 20 µl of additive B, and adding 200 µl of 20% (v/v) red cell solution. This analysis sample was placed in a 37°C water bath for five minutes, then injected into a Hemox Analyzer maintained at 37°C. The sample was completely deoxygenated by adding nitrogen through an adapter attached to the machine. Next, an oxygen dissociation curve was calculated while slowly injecting 20% oxygen (see Fig. 13).

The oxygen dissociation curve shows the degree of binding or dissociation of hemoglobin oxygen within red blood cells, based on dissolved oxygen partial pressure (pO₂). Fig. 13 shows the degree (%) of oxygen transport to tissues, the n value, or Hill coefficient indicating cooperative effect, and the P₅₀ value, which shows affinity with oxygen, all calculated from the oxygen dissociation curve in Fig. 13. As can be seen in Fig. 13, chemically modified red blood cells exhibit an oxygen dissociation curve form, P₅₀ value, n value, and oxygen transport to tissues similar to that of unmodified red blood cells. It was found, therefore, that mPEG bound to red blood cells does not cause a substantial change in oxygen transport activity.

The methology used in the following example is identical to that used in example 1 above but other functionalized polyethylene glycols (or derivatives thereof) are used.
This example establishes the general principle of the antigenic modulation of cells by the covalent linkage of nonimmunogenic materials.

The compounds used are as follows:

| | |
|---|---|
| Methoxypolyethylene glycol-cyanuric chloride | Molecular weight of 5,000 |
| Polyoxyethylene bis (imidazolyl carbonyl) | Molecular weight of 20,000 |
| Polyoxyethylene bis (p-nitrophenyl carbonate) | Molecular weight of 3,350 |

### EXAMPLE XIV

### Derivatization of Erythrocytes:

Whole blood (human, mouse, sheep, rat etc.) is obtained and washed 3x in isotonic saline. A red cell suspension (hematocrit of - 12%) is prepared in isotonic alkaline phosphate buffer (PBS; 50 mM K₂HPO₄ and 105 mM NaCl, pH - 9.2). The functionalized derivatives of either polyethylene oxide or polyoxethylene are added and the red erythrocytes are incubated for 30 minutes at 4°C. 3 mg of the functionalized derivatives was added to 1 ml of a 12% hematocrit in PBS (as indicated above). Following derivatization the covalently modified erythrocytes were washed thrice and erythrocyte microaggregation was measured using a platelet aggregometer (as with the methoxypolyethylene glycol-cyanuric chloride modified cells). Similiar procedures, though at lower pH (e.g., pH 8.0), have also been used to modify nucleated (epithelial and endothelial) cells. Indeed, the pH, time and temperature conditions at which the modification reactions can be done (based on the linker, e.g; cyanuric chloride versus nitrophenyl carbonates) are very malleable, thus making this invention applicable to a wide variety of cell types.
Importantly, in all of the above cases, the covalent modification of intact RBC with nonimmunogenic materials of various sizes (molecular weights of 3350, 5000 and 20000), using a wide variety of linkages, demonstrate decreased antigenic recognition of the cells (as demonstrated by decreased RBC agglutination in response to blood typing sera).

As was described in detail and demonstrated above, the present invention provides a method of producing red blood cells, in which agglutination caused by antigen-antibody reactions is suppressed. The access of blood group and tissue-specific antibodies is sterically obstructed through chemical modification by the covalent bonding of nontoxic polymers of mPEG in the area around blood group and tissue-specific antigens on the surface of red blood cells. Red blood cells chemically modified in accordance with the present invention, therefore, can be used regardless of blood type and have sufficient oxygen transport activity. As a result, they can be used in all medical fields in which red blood cells are used, for such purposes as emergency blood transfusion and transplant organ storage. The method of producing red blood cells chemically modified in accordance with the present invention can be applied to all blood group antigens regardless of blood type. The method is very simple and economical, making it very advantageous for the design of reactors for mass production in the future.

The covalent modification of the external cell membrane with non-immunogenic materials (e.g., mPEG) effectively "hides" both major and minor antigenic determinants on a large variety of nucleated and anucleated cells. The covalent attachment of non-immunogenic materials to intact cells (e.g., RBC, endothelial cells, epithelial cells, pancreatic β cells, etc.) can also be used for: (1) Derivatization, by perfusion of mPEG solutions, of human organ grafts to prevent unexpected hyperacute rejection episodes; and (2) Derivatization - by perfusion of mPEG solutions - of organs from non-human animals to prevent hyperacute rejection and to improve the chances of ultimate successful engraftment.

Although preferred embodiments have been depicted and described in detail herein, it will be apparent to those skilled in the relevant art that various modifications, additions, substitutions and the like can be made without departing from the spirit of the invention and these are therefore considered to be within the scope of the invention as defined in the claims which follow.

### LIST OF REFERENCES CITED

Abuchowslti, A. et al. (1977a) Alteration of immunological properties of bovine serum albumin by covalent attachment of polyethylene glycol. J. Biol. Chem., 252:3358-3581.
Abuchowski, A. et al. (1977b) Effect of covalent attachment of polyethylene glycol on immunogenicity and circulating life of bovine liver catalase. J. Biol. Chem., 252:33823586.
Harris, J. M. et al. (1984) Synthesis and characterization of Poly(ethylene Glycol) Derivatives. J. Poly. Sci., 22:341:352.
Harris, J. M. (1985) Laboratory Synthesis of Polyethylene Glycol Derivatives. Journal of Macromolecular Sciences Reviews in Macromolecular chemistry and Physics, C25:325-373.
Jackson, C-J. et al. (1987) Synthesis, isolation, and characterization of conjugates of ovalbumin with monomethoxypolyethylene glycol using cyanuric chloride as the coupling agent. Anal. Biochem., 165:114-127.
Klibanov, A. L. et al. (1991) Activity of amphipathic poly(ethylene glycol) 5000 to prolong the circulation time of liposomes depends on the liposome size and is unfavorable for immunoliposome binding to target. Biochim. Biophys. Acta, 1062:2782-1794.
Lacy, P. E. et al. (1991) Maintenance of Normoglycemia in Diabetic Mice by Subcutaneous Xenografts of Encapsulated Islets. Science, 254:1782-1794.
Lasic, D. (1992) Liposomes. American Scientist, 80:20-31.
Lim, F., and Sun, A. (1980) Microencapsulated Islets as bioartificial endocrine Pancreas. Science, 210:908-910.
Maruyama, K. et al. (1992) Prolonged circulation time *in vivo* of large unilamellar liposomes composed of distearoyl phosphatidylcholine and cholesterol containing amphipathic poly(ethylene glycol). Biochim. Biophys. Acta, 1128:44-49.
Merrill, E. W. Poly(Ethylene Oxide) and blood contact: A chronicle of one laboratory. *In:* Poly(Ethylene Glycol) Chemistry: Biotechnical and Biomedical Application (Harris, J. M., Editor) 1992, Plenum Press, N.Y., pp. 199-220.
Mitz, M. A. and Summaria L. J. (1961) Synthesis of biologically active cellulose derivatives of enzymes. Nature, 189:576-577.
Park, K. D. et al. PEO-Modified Surfaces - *In vitro*, *Ex vivo* and *In vivo* blood compatibility. *In:* Poly(Ethylene Glycol) Chemistry: Biotechnical and Biomedical Application (Harris, J. M., editor) 1992, Plenum Press, N.Y., pp. 283-302.
Sawhney, A. S. et al. (1994) Modification of Islet of Langerhans surfaces with immunoprotective poly(ethylene glycol) coatings via interfacial photopolymerization. Biotech. Bioeng., 44:383-386.
Senior, J. et al. (1991) Influence of surface hydrophilicity of liposomes on their interaction with plasma protein and clearance from the circulation: studies with poly(ethylene glycol)-coated vesicles. Biochim. Biophys. Acta, 1062:77-82.
Vichinsky, E. P. et al. (1990) Alloimmunization in sickle cell anemia and transfusion of racially unmatched blood. New Eng. J. Med., 322:1617-1621.
von Specht, B.-U. et al. (1973) Hoppe-Seyler's Z. Physiol. Chem., 354:1659-1660.
Zalipsky, S. and Lee, C. Use of functionalized Poly(Ethylene Glycol)s for modification of polypeptides. *In:* Poly(Ethylene Glycol) Chemistry: Biotechnical and Biomedical Application (Harris, J. M., editor) 1992, Plenum Press, N.Y., pp. 347-370.
Poly(Ethylene Glycol) Chemistry: Biotechnical and Biomedical Application Harris, J. M., editor (1992), Plenum Press, NY.

## Claims

1. A non-immunogenic cellular composition or cell (hereinafter referred to as "the cellular composition") comprising:
a mammalian or other cell having a cell surface and antigenic determinants on said cell surface;
a non-immunogenic compound covalently attached to said cell surface directly or by means of linking moieties, so that recognition of said antigenic determinants on said cell surface (or the antigenic nature of the cell surface) is blocked by said non-immunogenic compound.

2. The cellular composition of claim 1 wherein said non-immunogenic compound is a polyalkylene glycol.

3. The cellular composition of claim 2 wherein said non-immunogenic compound is methoxypolyethylene glycol.

4. The cellular composition of claim 1 wherein said non-immunogenic compound is dextran, Ficoll or arabinogalactan.

5. The cellular composition of claim 1 wherein said cell is a red blood cell.

6. The cellular composition of claim 5 wherein the antigenic determinants comprise a blood group antigenic determinant.

7. The cellular composition of claim 1 wherein said cell is a vascular endothelial cell, a hepatic cell, a neuronal cell, a pancreatic cell or an epithelial cell.

8. A method of rendering a mammalian or other cell having an immunogenic surface non-immunogenic, said method comprising:
covalently attaching a non-immunogenic compound to the cell surface, directly or by means of a linking moiety, so that said non-immunogenic compound blocks recognition of antigenic determinants on the cell surface (or the antigenic nature of the cell surface).

9. The method of claim 8 wherein said linking moiety is covalently attached to said antigenic determinants on said cell surface or to the cell surface other than through the antigenic determinants.

10. The method of claim 8 or 9 wherein said cell is a red blood cell.

11. A non-immunogenic cell produced by the method of claim 10, for transfusing a human.

12. The method of claim 8 or 9 wherein said cell is part of a tissue or organ.

13. The method of claim 12 wherein said cell is a vascular endothelial cell forming an exposed antigenic surface of the tissue or organ.

14. A mammalian cell for introduction into a mammal, said cell having a cell surface and antigenic determinants on said cell surface, the cell having been treated by a method comprising:
covalently attaching a non-immunogenic compound to said cell surface, directly or by means of a linking moiety, so that said non-immunogenic compound blocks recognition of said antigenic determinants on said cell surface (or the antigenic nature of the cell surface) and renders the cell non-immunogenic;
phagocytosis, complement-mediated destruction or cell-mediated destruction of the resulting non-immunogenic cell when introduced into the mammal being reduced.

15. A red blood cell for transfusing a mammal, said red blood cell having a cell surface and blood group antigenic determinants on said cell surface, said cell having been treated by a method comprising:
covalently attaching a non-immunogenic compound to said cell surface, directly or by means of a linking moiety, so that said non-immunogenic compound blocks recognition of said blood group antigenic determinants on said cell surface (or the antigenic nature of the cell surface) and produces a non-immunogenic red blood cell;
adverse reaction to transfusion of the non-immunogenic red blood cell when transfused into a mammal being decreased.

16. A mammalian cell for transplanting into a mammal, said cell having a cell surface and antigenic determinants on said cell surface, said cell having been treated by a method comprising:
covalently attaching an amount of a non-immunogenic compound to said cell surface, directly or by means of a linking moiety, so that said non-immunogenic compound blocks recognition of said antigenic determinants on said cell surface (or the antigenic nature of the cell surface);
rejection of the resulting non-immunogenic cell when transplanted into a mammal cell being decreased.

17. A method of decreasing antibody-induced aggregation of mammalian cells having cell surfaces, said method comprising:
covalently attaching a non-immunogenic compound to the cell surface of each of a population of cells, directly or by means of linking moieties, so that said non-immunogenic compound blocks recognition of antigenic determinants on the cell surface (or the antigenic nature of the cell surface), so as to produce non-aggregating cells, wherein antibody-induced aggregation of said non-aggregating cells is decreased as compared to antibody-induced aggregation of said cells prior to attachment of the compound.

## Patentansprüche

1. Immunogenfreie zellulare Zusammensetzung bzw. Zelle (nachfolgend als "zellulare Zusammensetzung" bezeichnet), aufweisend:
eine Säugetierzelle oder eine andere Zelle mit einer Zellenoberfläche und Antigen-Bestimmungsmitteln) auf der Zellenoberfläche;
eine immunogenfreie Zusammensetzung, die mit der Zellenoberfläche kovalent direkt oder mittels Verknüpfungskomponenten verbunden ist, so dass eine Erkennung der Antigen-Bestimmungsmittel auf der Zellenoberfläche (bzw. der antigenen Natur der Zellenoberfläche) durch die immunogenfreie Verbindung blockiert ist.

2. Zellulare Zusammensetzung nach Anspruch 1, wobei die immunogenfreie Verbindung ein Polyalkylenglykol ist.

3. Zellulare Zusammensetzung nach Anspruch 2, wobei die immunogenfreie Verbindung ein Methoxypolyethylenglykol ist.

4. Zellulare Zusammensetzung nach Anspruch 1, wobei die immunogenfreie Verbindung Dextran, Ficoll oder Arabinogalactan ist.

5. Zellulare Zusammensetzung nach Anspruch 1, wobei die Zelle ein rotes Blutkörperchen ist.

6. Zelluläre Zusammensetzung nach Anspruch 5, wobei die Antigen-Bestimmungsmittel ein Antigen-Blutgruppen-Bestimmungsmittel umfassen.

7. Zellulare Zusammensetzung nach Anspruch 1, wobei die genannte Zelle eine vaskulare Endothelzelle, eine Leberzelle, eine Neuronalzelle, eine Pankreaszelle oder eine Epithelzelle ist.

8. Verfahren zum Immunogenfreimachen eines Säugetiers oder einer Säugetierzelle mit einer immunogenen Oberfläche, wobei das Verfahren aufweist:
kovalentes Verbinden einer immunogenfreien Verbindung mit der Zellenoberfläche direkt oder mittels einer Verknüpfungskomponente, so dass die immunogenfreie Verbindung die Erkennung von Antigen-Bestimmungsmitteln auf der Zellenoberfläche (oder der antigenen Natur der Zellenoberfläche) blockiert.

9. Verfahren nach Anspruch 8, wobei die Verknüpfungskomponente mit dem Antigen-Bestimmungsmittel auf der Zellenoberfläche kovalent oder mit einer anderen Zellenoberfläche durch die Antigen-Bestimmungsmittel verbunden wird.

10. Verfahren nach Anspruch 8 oder 9, wobei die Zelle ein rotes Blutkörperchen ist.

11. Immunogenfreie Zelle, hergestellt durch das Verfahren nach Anspruch 10 zur Transfusion in eine menschliche Person.

12. Verfahren nach Anspruch 8 oder 9, wobei die Zelle einen Teil eines Gewebes oder eines Organs ist.

13. Verfahren nach Anspruch 12, wobei die Zelle eine vaskulare Endothelzelle ist, die eine freiliegende Antigen-Oberfläche des Gewebes oder des Organs bildet.

14. Säugetierzelle zum Einbau in ein Säugetier, wobei die Zelle eine Zellenoberfläche und Antigen-Bestimmungsmittel auf der Zellenoberfläche aufweist, wobei die Zelle durch ein Verfahren behandelt worden ist, das die Schritte aufweist:
kovalentes Verbinden einer immunogenfreien Verbindung mit der Zellenoberfläche direkt oder mittels einer Verknüpfungskomponente, so dass die imtnunogenfreie Verbindung die Erkennung der Antigen-Bestimmungsmittel auf der Zellenoberfläche (oder der antigenen Natur der Zellenoberfläche) blockiert und die Zelle immunogenfrei macht;
wobei Phagozytose, eine komplementvermittelte Zerstörung oder eine zellenvermittelte Zerstörung der resultierenden immunogenfreien Zelle reduziert werden, wenn diese in das Säugetier eingebracht wird.

15. Rotes Blutkörperchen zum Transfundieren in ein Säugetier, wobei das rote Blutkörperchen eine Zellenoberfläche und Blutgruppen- Antigen-Bestimmungsmittel auf der Zellenoberfläche aufweist, wobei die Zelle durch ein Verfahren behandelt worden ist, das die Schritte aufweist:
kovalentes Verbinden einer immunogenfreien Verbindung mit der Zellenoberfläche direkt oder mittels einer Verknüpfungskomponente, so dass die immunogenfreie Verbindung die Erkennung der Blutgruppen- Antigen-Bestimmungsmittel auf der Zellenoberfläche (oder der antigenen Natur der Zellenoberfläche) blockiert, und ein immunogenfreies rotes Blutkörperchen erzeugt;
wobei eine ungünstige Reaktion auf das Transfundieren des immunogenfreien roten Blutkörperchens verringert wird, wenn dieses in das Säugetier transfundiert wird.

16. Säugetierzelle zum Transplantieren in ein Säugetier, wobei die Zelle eine Zellenoberfläche und Antigen-Bestimmungsmittel auf der Zellenoberfläche aufweist, wobei die Zelle durch ein Verfahren behandelt worden ist, das die Schritte aufweist:
kovalentes Verbinden einer Menge einer immunogenfreien Verbindung mit der Zellenoberfläche direkt oder mittels einer Verknüpfungskomponente, so dass die immunogenfreie Verbindung die Erkennung der Antigen-Bestimmungsmittel auf der Zellenoberfläche (oder der antigenen Natur der Zellenoberfläche) blockiert;
wobei ein Abstoßen der resultierenden immunogenfreien Zelle verringert wird, wenn diese in die Säugetierzelle transplantiert wird.

17. Verfahren zum Verringern der Antikörper-induzierten Aggregation von Säugetierzellen mit Zellenoberflächen, wobei das Verfahren die Schritte aufweist:
kovalentes Verbinden einer immunogenfreien Verbindung mit der Zellenoberfläche von jeweils einer Population von Zellen direkt oder mittels Verknüpfungskomponenten, so dass die immunogenfreie Verbindung die Erkennung von Antigen-Bestimmungsmittel der Zellenoberfläche (oder der antigenen Natur der Zellenoberfläche) blockiert, um nicht-aggregierende Zellen zu erzeugen, wobei die Antikörper-induzierte Aggregation der nicht-aggregierenden Zellen im Vergleich zu einer Antikörper-induzierten Aggregation von Zellen vor dem Verbinden mit der Verbindung verringert wird.

## Revendications

1. Composition cellulaire ou cellule non immunogène (à laquelle il est ci-après fait référence comme à la "composition cellulaire") comprenant :
une cellule mammifère ou autre ayant une surface cellulaire et des déterminants antigéniques sur ladite surface cellulaire ;
un composé non immunogène lié par covalence à ladite surface cellulaire, directement ou aux moyens de groupes de liaison, de manière à ce que la reconnaissance desdits déterminants antigéniques sur ladite surface cellulaire (ou la nature antigénique de la surface cellulaire) soit bloquée par ledit composé non immunogène.

2. Composition cellulaire selon la revendication 1, dans laquelle ledit composé non immunogène est un polyalkylèneglycol.

3. Composition cellulaire selon la revendication 2, dans laquelle ledit composé non immunogène est un méthoxypolyéthylèneglycol.

4. Composition cellulaire selon la revendication 1, dans laquelle ledit composé non immunogène est le dextrane, le Ficoll ou l'arabinogalactane.

5. Composition cellulaire selon la revendication 1, dans laquelle ladite cellule est un globule rouge.

6. Composition cellulaire selon la revendication 5, dans laquelle les déterminants antigéniques comprennent un déterminant antigénique du groupe sanguin.

7. Composition cellulaire selon la revendication 1, dans laquelle ladite cellule est une cellule endothéliale vasculaire, une cellule hépatique, une cellule neuronale, une cellule pancréatique ou une cellule épithéliale.

8. Procédé destiné à rendre non immunogène une cellule mammifère ou autre ayant une surface immunogène, ledit procédé comprenant :
la liaison par covalence d'un composé non immunogène à la surface cellulaire, directement ou au moyen d'un groupe de liaison, de manière à ce que ledit composé non immunogène bloque la reconnaissance des déterminants antigéniques sur la surface cellulaire (ou la nature antigénique de la surface cellulaire).

9. Procédé selon la revendication 8, dans lequel ledit groupe de liaison est lié par covalence auxdits déterminants antigéniques sur ladite surface cellulaire ou à la surface cellulaire de manière autre que par les déterminants antigéniques.

10. Procédé selon la revendication 8 ou 9, dans lequel ladite cellule est un globule rouge.

11. Cellule non immunogène produite par le procédé selon la revendication 10, destinée à être transfusée à un humain.

12. Procédé selon la revendication 8 ou 9, dans lequel ladite cellule fait partie d'un tissu ou d'un organe.

13. Procédé selon la revendication 12, dans lequel ladite cellule est une cellule endothéliale vasculaire formant une surface antigénique exposée du tissu ou de l'organe.

14. Cellule mammifère destinée à être introduite dans un mammifère, ladite cellule ayant une surface cellulaire et des déterminants antigéniques sur ladite surface cellulaire, la cellule ayant été traitée par un procédé comprenant :
la liaison par covalence d'un composé non immunogène à ladite surface cellulaire, directement ou au moyen d'un groupe de liaison, de manière à ce que ledit composé non immunogène bloque la reconnaissance desdits déterminants antigéniques sur ladite surface cellulaire (ou la nature antigénique de la surface cellulaire) et rende la cellule non immunogène ;
la phagocytose, la destruction médiée par le complément ou la destruction médiée par les cellules de la cellule non immunogène obtenue, lorsqu'elle est introduite dans le mammifère, étant réduite.

15. Globule rouge destiné à être transfusé à un mammifère, ledit globule rouge ayant une surface cellulaire et des déterminants antigéniques du groupe sanguin sur ladite surface cellulaire, ledit globule ayant été traité par un procédé comprenant :
la liaison par covalence d'un composé non immunogène à ladite surface cellulaire, directement ou au moyen d'un groupe de liaison, de manière à ce que ledit composé non immunogène bloque la reconnaissance desdits déterminants antigéniques du groupe sanguin sur ladite surface cellulaire (ou la nature antigénique de la surface cellulaire) et produise un globule rouge non immunogène ;
la réaction adverse à la transfusion du globule rouge non immunogène, lorsqu'il est transfusé à un mammifère, étant réduite.

16. Cellule mammifère destinée à être transplantée dans un mammifère, ladite cellule ayant une surface cellulaire et des déterminants antigéniques sur ladite surface cellulaire, ladite cellule ayant été traitée par un procédé comprenant :
la liaison par covalence d'une quantité d'un composé non immunogène à ladite surface cellulaire, directement ou au moyen d'un groupe de liaison, de manière à ce que ledit composé non immunogène bloque la reconnaissance desdits déterminants antigéniques sur ladite surface cellulaire (ou la nature antigénique de la surface cellulaire) ;
le rejet de la cellule non immunogène obtenue, lorsqu'elle est transplantée dans une cellule mammifère, étant réduit.

17. Procédé de diminution de l'agrégation induite par les anticorps des cellules mammifères ayant des surfaces cellulaires, ledit procédé comprenant :
la liaison par covalence d'un composé non immunogène à la surface cellulaire de chacune des cellules d'une population de cellules, directement ou aux moyens de groupes de liaison, de manière à ce que ledit composé non immunogène bloque la reconnaissance des déterminants antigéniques sur la surface cellulaire (ou la nature antigénique de la surface cellulaire), de manière à produire des cellules ne s'agrégeant pas, dans lequel l'agrégation induite par les anticorps desdites cellules ne s'agrégeant pas est réduite comparée à l'agrégation induite par les anticorps desdites cellules avant la liaison du composé.
